# EUROPEAN PATENT SPECIFICATION

(11) **EP 3 925 536 B1**
(45) Date of publication and mention of the grant of the patent: **11.10.2023**
(21) Application number: 21180309.3
(22) Date of filing: 18.06.2021
(51) Int. Cl.: A61B 5/367, A61B 5/346, A61B 5/00

(54) **VENTRICULAR FAR FIELD ESTIMATION USING AUTOENCODER**
VENTRIKULÄRE FERNFELDSCHÄTZUNG UNTER VERWENDUNG EINES AUTOCODIERERS
ESTIMATION DE CHAMP LOINTAIN VENTRICULAIRE UTILISANT UN AUTO-ENCODEUR

(30) Priority: 19.06.2020 US 202063041495 P; 30.06.2020 US 202063046295 P; 16.06.2021 US 202117349564
(43) Date of publication of application: 22.12.2021
(73) Proprietor: Biosense Webster (Israel) Ltd, Yokneam, 2066717 (IL)
(72) Inventor: Amos, Yariv Avraham, Yokneam (IL); Goldberg, Stanislav, Yokneam (IL); Amit, Matityahu, Yokneam (IL); Bar-Tal, Meir, Yokneam (IL); Malki, Guy David, Yokneam (IL); Tsoref, Liat, Yokneam (IL)
(74) Representative: Carpmaels & Ransford LLP

(56) References cited:
- EP-A2- 3 928 701
- US-A1- 2016 175 023
- XIONG PENG ET AL: "ECG signal enhancement based on improved denoising auto-encoder", ENGINEERING APPLICATIONS OF ARTIFICIAL INTELLIGENCE, PINERIDGE PRESS, SWANSEA, GB, vol. 52, 6 May 2016 (2016-05-06), pages 194-202, XP029530661, ISSN: 0952-1976, DOI: 10.1016/J.ENGAPPAI.2016.02.015

## Description

### FIELD OF INVENTION

The present invention is related to an artificial intelligence and machine learning autoencoder associated with ventricular far field estimation, and identification and decomposition of near and far field signals in cardiac electrical activity.

### BACKGROUND

Treatments for cardiac conditions, such as cardiac arrhythmia, often require heart mapping (i.e., mapping cardiac tissue, chambers, veins, arteries and/or pathways, which is also known as cardiac mapping). Electrocardiograms or electrocardiographs (ECGs) are examples of heart mapping. ECGs are generated from electrical signals from a heart that describe heart activity.

ECGs are utilized during cardiac procedures to identify potential origination locations of cardiac conditions. Generally, when physicians use ECGs to study heart activity, signal interference, signal artifacts, and signal noise associated with the underlying electrical signals of the ECGs can particularly obscure the accuracy of the ECGs. Signal interference may also result from the processing of areas of the signal with sharp changes, peaks, and/or pacing signals including areas of high frequency and harmonics. Due to these interferences, artifacts, and noise, physicians are unable to separate ventricle and atria origination locations in real time cases (e.g., during cardiac procedures), which add difficulties to diagnosing/treating cardiac conditions. Therefore, a need exists to provide improved methods for heart mapping that removes such interferences, artifacts, and noise.

Unipolar signals are a combination of near and far field signals. During an ablation procedure it is important to identify and isolate the near field signal. When electrodes are inserted into a muscle, such as a heart muscle, each activation of the muscle generates an electrical field. Each electrode captures all sources of the electric field in the location it is placed, including the near field signal near the electrode and the far field signal farther from the electrode.
In XIONG PENG ET AL, "ECG signal enhancement based on improved denoising auto-encoder", ENGINEERING APPLICATIONS OF ARTIFICIAL INTELLIGENCE, PINERIDGE PRESS, SWANSEA, GB, (20160506), vol. 52, doi:10.1016/J.ENGAPPAI.2016.02.015, ISSN 0952-1976, pages 194 - 202, there is described a deep neural network which is proposed to solve the problem of noise contaminating ECG signals. The DNN is created from an improved denoising auto-encoder (DAE) reformed by a wavelet transform (WT) method. A WT with scale-adaptive thresholding method is used to filter most of the noise. The DNN based on improved DAE is then used to remove any residual noise.
US 2016/175023 A1 describes far field reduction carried out in a cardiac electrogram by extracting unipolar beats of an intracardiac electrogram that occur within a predetermined time interval that includes QRS peaks, constructing a first mean unipolar beat by averaging the extracted unipolar beats, and accepting unipolar beats that cross-correlate with the first mean unipolar beat. A second mean unipolar beat is constructed from the accepted unipolar beats. A ventricular far field component is determined from the extracted unipolar beats and subtracted from the intracardiac electrogram to distinguish a local component of the intracardiac electrogram.

### SUMMARY

According to an embodiment, a method is provided. The method includes receiving input intracardiac signals from a monitoring and processing apparatus. Each of the input intracardiac signals may include at least artifacts. The method includes encoding, by an autoencoder, the input intracardiac signals utilizing an intracardiac dataset to produce a latent representation. The method also includes decoding, by an autoencoder, the latent representation to produce output intracardiac signals. The output intracardiac signals may include the input intracardiac signals reconstructed without the artifacts.

A method in accordance with the invention is defined in claim 1. A system in accordance with the invention is defined in claim 6.

### BRIEF DESCRIPTION OF THE DRAWINGS

A more detailed understanding may be had from the following description, given by way of example in conjunction with the accompanying drawings, wherein like reference numerals in the figures indicate like elements, and wherein:
FIG. 1 illustrates a diagram of an exemplary system in which one or more features of the disclosure subject matter can be implemented according to one or more embodiments;
FIG. 2 illustrates a block diagram of an example system for remotely monitoring and communicating patient biometrics according to one or more embodiments;
FIG. 3 illustrates a graphical depiction of an artificial intelligence system according to one or more embodiments;
FIG. 4 illustrates a block diagram of a method performed in the artificial intelligence system of FIG. 3 according to one or more embodiments;
FIG. 5 illustrates an example of a neural network according to one or more embodiments;
FIG. 6 illustrates a block diagram of a method according to one or more embodiments;
FIG. 7 illustrates a graphical depiction of a signal according to one or more embodiments;
FIG. 8 illustrates a graphical depiction of a signal according to one or more embodiments;
FIG. 9 illustrates a graphical depiction of a signal according to one or more embodiments;
FIG. 10 illustrates a graphical depiction of a signal progression according to one or more embodiments;
FIG. 11 illustrates a block diagram of a method according to one or more embodiments; and
FIG. 12 is an example flow diagram of an exemplary method of decomposing near and far field signals in accordance with an embodiment.

### DETAILED DESCRIPTION

Disclosed herein is an artificial intelligence and machine learning autoencoder (generally referred herein as an autoencoder). The autoencoder may be a processor executable code or software that is necessarily rooted in process operations by, and in processing hardware of, medical device equipment to provide improved ECGs for treating cardiac conditions. According to an embodiment, the autoencoder may provide a specific encoding and decoding method for the medical device equipment. This specific encoding and decoding method may involve a multi-step data manipulation of electrical signals of a heart that removes signal interference, signal artifacts, and signal noise from the electrical signals.

In this regard and in operation, the autoencoder may receive input intracardiac signals (e.g., the electrical signals of the heart that include signal interference, signal artifacts, and signal noise). The intracardiac signals may be, in real time, recorded and processed by a monitoring and processing apparatus (e.g., a catheter with the autoencoder therein) and/or recorded and transmitted by the monitoring and processing apparatus to a computing device with the autoencoder therein.

The autoencoder may encode the input intracardiac signals utilizing an intracardiac dataset (e.g., predetermined and approved electrical signals of the heart that are free from signal interference, signal artifacts, and signal noise). This encoding by the autoencoder may produce a latent representation from the input intracardiac signals. The autoencoder may further decode the latent representation to produce output intracardiac signals. The output intracardiac signals may be the input intracardiac signals reconstructed without the signal interference, signal artifacts, and signal noise. The improved ECGs for treating cardiac conditions are then generated from the output intracardiac signals.

The technical effects of the autoencoder include producing the output intracardiac signals, in real time, which further enable the generation of the improved ECGs to physicians (such as during cardiac procedures) who use the improved ECGs to study heart activity to identify potential origination locations of cardiac conditions. The improved ECGs are not obscured by the signal interference, the signal artifacts, and the signal noise of the original input intracardiac signals, as these artifacts have been removed during decoding. Further, the technical effects of the autoencoder include producing the improved ECGs with an increased accuracy, with the signal interference, signal artifacts, and signal noise removed, to allow the ventricle and atria origination locations to be separately provided in real time.

In an embodiment, an autoencoder may be utilized to train a system to decompose near and far field signals detected by electrodes from analyzing a large number of data points. Bits may be selected as part of a training set, which are used to train the system to recognize the far field signal component.

A signal may be provided and a regeneration of the signal may be attempted, by providing signals with a large amount of far field signals and a large amount with both far and near field signals. The autoencoder may be provided a signal to reconstruct the far field signal. Once the network is trained, the far field component may be output from a provided signal by the network.

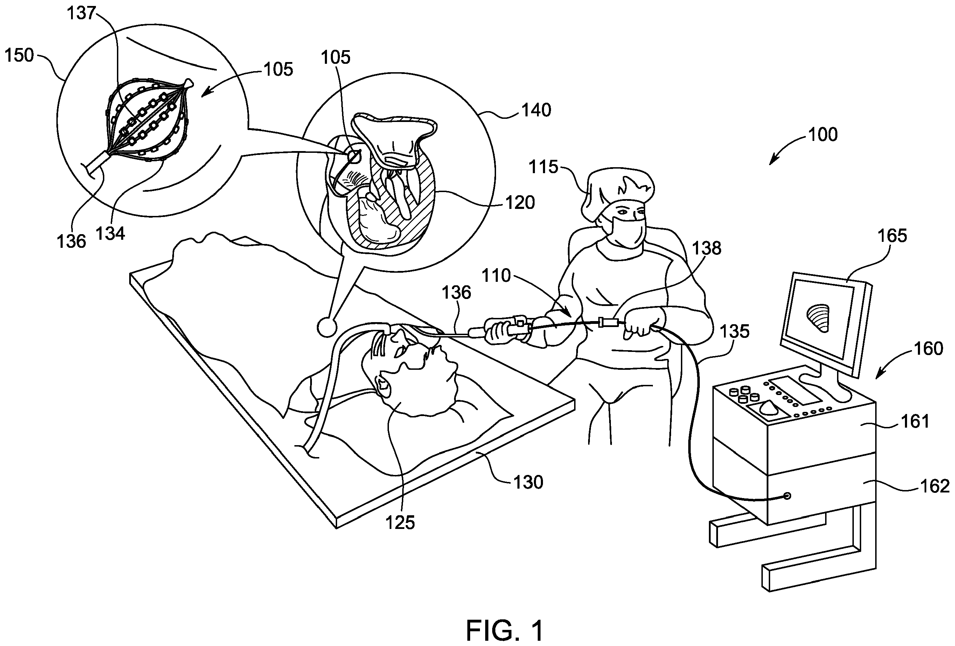

FIG. 1 is a diagram of an exemplary system 100 (e.g., medical device equipment) in which one or more features of the disclosure subject matter can be implemented. All or parts of system 100 may be used to collect information for an intracardiac dataset (e.g., a training dataset) and/or all or parts of system 100 may be used to implement the autoencoder described herein (e.g., a trained model).

The system 100 may include components, such as a catheter 105, that are configured to damage tissue areas of an intra-body organ. The catheter 105 may also be further configured to obtain biometric data including the electrical signals of the heart (e.g., the intracardiac signals). Although the catheter 105 is shown to be a point catheter, it will be understood that a catheter of any shape that includes one or more elements (e.g., electrodes) may be used to implement the embodiments disclosed herein.

The system 100 includes a probe 110, having shafts that may be navigated by a physician or a medical professional 115 into a body part, such as a heart 120, of a patient 125 lying on a bed (or a table) 130. According to embodiments, multiple probes may be provided; however, for purposes of conciseness, a single probe 110 is described herein. Yet, it is understood that the probe 110 may represent multiple probes.

The exemplary system 100 can be utilized to detect, diagnose, and treat cardiac conditions (e.g., using the intracardiac signals). Cardiac conditions, such as cardiac arrhythmias (atrial fibrillation in particular), persist as common and dangerous medical ailments, especially in the aging population. In patients (e.g., the patient 125) with normal sinus rhythm, the heart (e.g., the heart 120), which includes atrial, ventricular, and excitatory conduction tissue, is electrically excited to beat in a synchronous, patterned fashion. This electrical excitement may be detected as intracardiac signals.

In patients (e.g., the patient 125) with cardiac arrhythmias, abnormal regions of cardiac tissue do not follow the synchronous beating cycle associated with normally conductive tissue as in patients with normal sinus rhythm. Instead, the abnormal regions of cardiac tissue aberrantly conduct to adjacent tissue, thereby disrupting the cardiac cycle into an asynchronous cardiac rhythm. This asynchronous cardiac rhythm can also be detected as intracardiac signals. Such abnormal conduction has been previously known to occur at various regions of the heart (e.g., the heart 120), for example, in the region of the sino-atrial (SA) node, along the conduction pathways of the atrioventricular (AV) node, or in the cardiac muscle tissue forming the walls of the ventricular and atrial cardiac chambers.

Further, cardiac arrhythmias, including atrial arrhythmias, may be of a multiwavelet reentrant type, characterized by multiple asynchronous loops of electrical impulses that are scattered about the atrial chamber and are often self-propagating (e.g., another example of intracardiac signals). Alternatively, or in addition to the multiwavelet reentrant type, cardiac arrhythmias may also have a focal origin, such as when an isolated region of tissue in an atrium fires autonomously in a rapid, repetitive fashion (e.g., another example of the intracardiac signals). Ventricular tachycardia (V-tach or VT) is a tachycardia, or fast heart rhythm that originates in one of the ventricles of the heart. This is a potentially life-threatening arrhythmia because it may lead to ventricular fibrillation and sudden death.

One type of arrhythmia, atrial fibrillation, occurs when the normal electrical impulses (e.g., another example of the intracardiac signals) generated by the sinoatrial node are overwhelmed by disorganized electrical impulses (e.g., the signal interference) that originate in the atria and pulmonary veins causing irregular impulses to be conducted to the ventricles. An irregular heartbeat results and may last from minutes to weeks, or even years. Atrial fibrillation (AF) is often a chronic condition that leads to a small increase in the risk of death often due to strokes. The first line of treatment for AF is medication that either slows the heart rate or revert the heart rhythm back to normal. Additionally, persons with AF are often given anticoagulants to protect them from the risk of stroke. The use of such anticoagulants comes with its own risk of internal bleeding. In some patients, medication is not sufficient and their AF is deemed to be drug-refractory, i.e., untreatable with standard pharmacological interventions. Synchronized electrical cardioversion may also be used to convert AF to a normal heart rhythm. Alternatively, AF patients are treated by catheter ablation.

A catheter ablation-based treatment may include mapping the electrical properties of heart tissue, especially the endocardium and the heart volume, and selectively ablating cardiac tissue by application of energy. Cardiac mapping (e.g., heart mapping), for example, includes creating a map of electrical potentials (e.g., a voltage map) of the wave propagation along the heart tissue or a map of arrival times (e.g., a local time activation (LAT) map) to various tissue located points. Cardiac mapping may be used for detecting local heart tissue dysfunction. Ablations, such as those based on cardiac mapping, can cease or modify the propagation of unwanted electrical signals from one portion of the heart to another.

The ablation process damages the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure-mapping followed by ablation-electrical activity at points within the heart is typically sensed and measured by advancing a catheter (e.g., the catheter 105) containing one or more electrical sensors (e.g., the at least one ablation electrode 134 of the catheter 105) into the heart (e.g., the heart 120), and acquiring data at a multiplicity of points. This data (e.g., biometric data including the intracardiac signals) is then utilized to select the endocardial target areas, at which ablation is to be performed. Due to the use of the autoencoder employed by the exemplary system 100 (e.g., medical device equipment), this data is more accurate and better able to support selecting the endocardial target areas for ablation than the underlying electrical signals of the ECGs that include signal interference, signal artifacts, and signal noise. The signal interference, the signal artifacts, and the signal noise can be collectively referred to herein as artifacts. Examples of artifacts include, but are not limited to, power noise (e.g., electrostatic and electromagnetic coupling between the circuitry and 50 or 60 Hz power lines), Fuoro noise (e.g., fluorescent lights), contact noise (e.g., collision between catheter electrodes), and deflection noise (e.g., discharges of static electricity during catheter deflection).

Cardiac ablation and other cardiac electrophysiological procedures have become increasingly complex as clinicians treat challenging conditions such as atrial fibrillation and ventricular tachycardia. The treatment of complex arrhythmias can now rely on the use of three-dimensional (3D) mapping systems in order to reconstruct the anatomy of the heart chamber of interest. In this regard, the autoencoder employed by the exemplary system 100 (e.g., medical device equipment) herein provides the underlying output signals so that improved 3D maps and/or ECGs for treating cardiac conditions can be generated.

For example, cardiologists rely upon software, such as the Complex Fractionated Atrial Electrograms (CFAE) module of the CARTO^{®} 3 3D mapping system, produced by Biosense Webster, Inc. (Diamond Bar, Calif.), to generate and analyze intracardiac electrograms (EGM). The autoencoder of the exemplary system 100 (e.g., medical device equipment) enhances this software to generate and analyze improved intracardiac electrograms (EGM) so that the ablation points can be determined for treatment of a broad range of cardiac conditions, including atypical atrial flutter and ventricular tachycardia.

The improved 3D maps supported by the autoencoder can provide multiple pieces of information regarding the electrophysiological properties of the tissue that represent the anatomical and functional substrate of these challenging arrhythmias.

Cardiomyopathies with different etiologies (ischemic, dilated cardiomyopathy (DCM), hypertrophic cardiomyopathy (HCM), arrhythmogenic right ventricular dysplasia (ARVD), left ventricular non-compaction (LVNC), etc.) have an identifiable substrate, featured by areas of unhealthy tissue surrounded by areas of normally functioning cardiomyocytes.

Abnormal tissue is generally characterized by low-voltage EGMs. However, initial clinical experience in endo-epicardial mapping indicates that areas of low-voltage are not always present as the sole arrhythmogenic mechanism in such patients. In fact, areas of low or medium voltage may exhibit EGM fragmentation and prolonged activities during sinus rhythm, which corresponds to the critical isthmus identified during sustained and organized ventricular arrhythmias, e.g., applies only to non-tolerated ventricular tachycardias. Moreover, in many cases, EGM fragmentation and prolonged activities are observed in the regions showing a normal or near-normal voltage amplitude (>1-1.5 mV). Although the latter areas may be evaluated according to the voltage amplitude, they cannot be considered as normal according to the intracardiac signal, thus representing a true arrhythmogenic substrate. The 3D mapping may be able to localize the arrhythmogenic substrate on the endocardial and/or epicardial layer of the right/left ventricle, which may vary in distribution according to the extension of the main disease.

The substrate linked to these cardiac conditions is related to the presence of fragmented and prolonged EGMs in the endocardial and/or epicardial layers of the ventricular chambers (right and left). The 3D mapping system, such as CARTO^{®} 3, is able to localize the potential arrhythmogenic substrate of the cardiomyopathy in terms of abnormal EGM detection.

Electrode catheters (e.g., the catheter 105) are use in medical practice. Electrode catheters are used to stimulate and map electrical activity in the heart and to ablate sites of aberrant electrical activity. In use, the electrode catheter is inserted into a major vein or artery, e.g., femoral artery, and then guided into the chamber of the heart of concern. A typical ablation procedure involves the insertion of a catheter having at least one electrode at its distal end, into a heart chamber. A reference electrode is provided, generally taped to the skin of the patient or by means of a second catheter that is positioned in or near the heart. Radio frequency (RF) current is applied to the tip electrode of the ablating catheter, and current flows through the media that surrounds it, i.e., blood and tissue, toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. During this process, heating of the electrode also occurs as a result of conduction from the heated tissue to the electrode itself. If the electrode temperature becomes sufficiently high, possibly above 60 degrees Celsius, a thin transparent coating of dehydrated blood protein can form on the surface of the electrode. If the temperature continues to rise, this dehydrated layer can become progressively thicker resulting in blood coagulation on the electrode surface. Because dehydrated biological material has a higher electrical resistance than endocardial tissue, impedance to the flow of electrical energy into the tissue also increases. If the impedance increases sufficiently, an impedance rise occurs, and the catheter must be removed from the body and the tip electrode cleaned.

Treatments for cardiac conditions such as cardiac arrhythmia often require obtaining a detailed mapping of cardiac tissue, chambers, veins, arteries and/or electrical pathways. For example, a prerequisite for performing a catheter ablation successfully is that the cause of the cardiac arrhythmia is accurately located in the heart chamber. Such locating may be done via an electrophysiological investigation during which electrical potentials are detected spatially resolved with a mapping catheter introduced into the heart chamber. This electrophysiological investigation, the so-called electro-anatomical mapping, thus provides 3D mapping data which can be displayed on a monitor. In many cases, the mapping function and a treatment function (e.g., ablation) are provided by a single catheter or group of catheters such that the mapping catheter also operates as a treatment (e.g., ablation) catheter at the same time. In this case, the autoencoder can be directly stored and executed by the catheter 105.

Mapping of cardiac areas such as cardiac regions, tissue, veins, arteries and/or electrical pathways of the heart (e.g., 120) may result in identifying problem areas such as scar tissue, arrhythmia sources (e.g., electric rotors), healthy areas, and the like. Cardiac areas may be mapped such that a visual rendering of the mapped cardiac areas is provided using a display, as further disclosed herein. Additionally, cardiac mapping may include mapping based on one or more modalities such as, but not limited to local activation time (LAT), an electrical activity, a topology, a bipolar mapping, a dominant frequency, or an impedance. Data corresponding to multiple modalities may be captured using a catheter inserted into a patient's body and may be provided for rendering at the same time or at different times based on corresponding settings and/or preferences of a medical professional.

Cardiac mapping may be implemented using one or more techniques. As an example of a first technique, cardiac mapping may be implemented by sensing an electrical property of heart tissue, for example, LAT, as a function of the precise location within the heart. The corresponding data may be acquired with one or more catheters that are advanced into the heart using catheters that have electrical and location sensors in their distal tips. As specific examples, location and electrical activity may be initially measured on about 10 to about 20 points on the interior surface of the heart. These data points may be generally sufficient to generate a preliminary reconstruction or map of the cardiac surface to a satisfactory quality. The preliminary map may be combined with data taken at additional points in order to generate a more comprehensive map of the heart's electrical activity. In clinical settings, it is not uncommon to accumulate data at 100 or more sites to generate a detailed, comprehensive map of heart chamber electrical activity. The generated detailed map may then serve as the basis for deciding on a therapeutic course of action, for example, tissue ablation, to alter the propagation of the heart's electrical activity and to restore normal heart rhythm.

Returning to FIG. 1, to implement the noted cardiac mapping, the medical professional 115 may insert a shaft 137 through a sheath 136, while manipulating a distal end of the shaft 137 using a manipulator 138 near the proximal end of the catheter 105 and/or deflection from the sheath 136. As shown in an inset 140, the catheter 105 may be fitted at the distal end of the shaft 137. The catheter 105 may be inserted through the sheath 136 in a collapsed state and may be then expanded within the heart 120. The catheter 105 may include at least one ablation electrode 134 and a catheter needle, as further disclosed herein.

According to embodiments, the catheter 105 may be configured to ablate tissue areas of a cardiac chamber of the heart 120. Inset 150 shows the catheter 105 in an enlarged view, inside a cardiac chamber of the heart 120. As shown, the catheter 105 may include the at least one ablation electrode 134 coupled onto the body of the catheter. According to other embodiments, multiple elements may be connected via splines that form the shape of the catheter 105. One or more other elements (not shown) may be provided and may be any elements configured to ablate or to obtain biometric data and may be electrodes, transducers, or one or more other elements.

According to embodiments disclosed herein, the ablation electrodes, such as the at least one ablation electrode 134, may be configured to provide energy to tissue areas of an intra-body organ such as heart 120. The energy may be thermal energy and may cause damage to the tissue area starting from the surface of the tissue area and extending into the thickness of the tissue area.

According to embodiments disclosed herein, biometric data may include one or more of LATs, electrical activity, topology, bipolar mapping, dominant frequency, impedance, or the like. The LAT may be a point in time of a threshold activity corresponding to a local activation, calculated based on a normalized initial starting point. Electrical activity may be any applicable electrical signals that may be measured based on one or more thresholds and may be sensed and/or augmented based on signal to noise ratios and/or other filters. A topology may correspond to the physical structure of a body part or a portion of a body part and may correspond to changes in the physical structure relative to different parts of the body part or relative to different body parts. A dominant frequency may be a frequency or a range of frequency that is prevalent at a portion of a body part and may be different in different portions of the same body part. For example, the dominant frequency of a pulmonary vein of a heart may be different than the dominant frequency of the right atrium of the same heart. Impedance may be the resistance measurement at a given area of a body part.

As shown in FIG. 1, the probe 110 and the catheter 105 may be connected to a console 160. The console 160 may include a computing device 161, which employs the autoencoder as described herein. According to an embodiment, the console 160 and/or the computing device 161 include at least a processor and a memory, where the processor executes computer instructions with respect the autoencoder described herein and the memory stores the instructions for execution by the processor.

The computing device 161 can be any computing device including software and/or hardware, such as a general-purpose computer, with suitable front end and interface circuits 162 for transmitting and receiving signals to and from the catheter 105, as well as for controlling the other components of system 100. The computing device 161 may include real-time noise reduction circuitry typically configured as a field programmable gate array (FPGA), followed by an analog-to-digital (A/D) electrocardiograph or electromyogram (EMG) signal conversion integrated circuit. The computing device 161 may pass the signal from an A/D ECG or EMG circuit to another processor and/or can be programmed to perform one or more functions disclosed herein.

For example, the one or more functions include receiving input intracardiac signals, encoding the input intracardiac signals utilizing an intracardiac dataset to produce a latent representation, and decoding the latent representation to produce output intracardiac signals. The front end and interface circuits 162 include input/output (I/O) communication interfaces that enables the console 160 to receive signals from and/or transfer signals to the at least one ablation electrode 134.

In some embodiments, the computing device 161 may be further configured to receive biometric data, such as electrical activity, and determine if a given tissue area conducts electricity. According to an embodiment, the computing device 161 may be external to the console 160 and may be located, for example, in the catheter, in an external device, in a mobile device, in a cloud-based device, or may be a standalone processor.

As noted above, the computing device 161 may include a general-purpose computer, which may be programmed in software to carry out the functions of the autoencoder described herein. The software may be downloaded to the general-purpose computer in electronic form, over a network, for example, or it may, alternatively or additionally, be provided and/or stored on non-transitory tangible media, such as magnetic, optical, or electronic memory (e.g., any suitable volatile and/or non-volatile memory, such as random-access memory or a hard disk drive). The example configuration shown in FIG. 1 may be modified to implement the embodiments disclosed herein. The disclosed embodiments may similarly be applied using other system components and settings. Additionally, system 100 may include additional components, such as elements for sensing electrical activity, wired or wireless connectors, processing and display devices, or the like.

According to an embodiment, a display 165 is connected to the computing device 161. During a procedure, the computing device 161 may facilitate the presentation of a body part rendering to the medical professional 115 on a display 165, and store data representing the body part rendering in a memory. In some embodiments, the medical professional 115 may be able to manipulate the body part rendering using one or more input devices such as a touch pad, a mouse, a keyboard, a gesture recognition apparatus, or the like. For example, an input device may be used to change a position of the catheter 105, such that rendering is updated. In alternative embodiments, the display 165 may include a touchscreen that can be configured to accept inputs from the medical professional 115, in addition to presenting the body part rendering. Display 165 may be located at a same location or a remote location such as a separate hospital or in separate healthcare provider networks. Additionally, the system 100 may be part of a surgical system that is configured to obtain anatomical and electrical measurements of a patient's organ, such as the heart 120, and performing a cardiac ablation procedure. An example of such a surgical system is the Carto^{®} system sold by Biosense Webster.

The console 160 may be connected, by a cable, to body surface electrodes, which may include adhesive skin patches that are affixed to the patient 125. The processor, in conjunction with a current tracking module, may determine position coordinates of the catheter 105 inside the body part (e.g., the heart 120) of the patient 125. The position coordinates may be based on impedances or electromagnetic fields measured between the body surface electrodes and the electrode or other electromagnetic components (e.g., the at least one ablation electrode 134) of the catheter 105. Additionally, or alternatively, location pads may be located on a surface of bed 130 and may be separate from the bed 130.

The system 100 may also, and optionally, obtain biometric data such as anatomical measurements of the heart 120 using ultrasound, computed tomography (CT), magnetic resonance imaging (MRI) or other medical imaging techniques known in the art. The system 100 may obtain ECGs or electrical measurements using catheters or other sensors that measure electrical properties of the heart 120. The biometric data including anatomical and electrical measurements may then be stored in a non-transitory tangible media of the console 160. The biometric data may be transmitted to the computing device 161 from the non-transitory tangible media. Alternatively, or in addition, the biometric data may be transmitted to a server, which may be local or remote, using a network as further described herein.

According to one or more embodiments, catheters containing position sensors may be used to determine the trajectory of points on the cardiac surface. These trajectories may be used to infer motion characteristics such as the contractility of the tissue. Maps depicting such motion characteristics may be constructed when the trajectory information is sampled at a sufficient number of points in the heart 120.

Electrical activity at a point in the heart 120 may be typically measured by advancing the catheter 105 containing an electrical sensor at or near its distal tip (e.g., the at least one ablation electrode 134) to that point in the heart 120, contacting the tissue with the sensor and acquiring data at that point. One drawback with mapping a cardiac chamber using the catheter 105 containing only a single, distal tip electrode is the long period of time required to accumulate data on a point-by-point basis over the requisite number of points required for a detailed map of the chamber as a whole. Accordingly, multiple-electrode catheters have been developed to simultaneously measure electrical activity at multiple points in the heart chamber.

Multiple-electrode catheters may be implemented using any applicable shape such as a linear catheter with multiple electrodes, a balloon catheter including electrodes dispersed on multiple spines that shape the balloon, a lasso or loop catheter with multiple electrodes, or any other applicable shape. Linear catheters may be fully or partially elastic such that it can twist, bend, and or otherwise change its shape based on received signal and/or based on application of an external force (e.g., cardiac tissue) on the linear catheter. The balloon catheter may be designed such that when deployed into a patient's body, its electrodes may be held in intimate contact against an endocardial surface. As an example, a balloon catheter may be inserted into a lumen, such as a pulmonary vein (PV). The balloon catheter may be inserted into the PV in a deflated state such that the balloon catheter does not occupy its maximum volume while being inserted into the PV. The balloon catheter may expand while inside the PV such those electrodes on the balloon catheter are in contact with an entire circular section of the PV. Such contact with an entire circular section of the PV, or any other lumen, may enable efficient mapping and/or ablation.

According to an example, a multi-electrode catheter may be advanced into a chamber of the heart 120. Anteroposterior (AP) and lateral fluorograms may be obtained to establish the position and orientation of each of the electrodes. EGMs may be recorded from each of the electrodes in contact with a cardiac surface relative to a temporal reference such as the onset of the P-wave in sinus rhythm from a body surface ECG. The system, as further disclosed herein, may differentiate between those electrodes that register electrical activity and those that do not due to absence of close proximity to the endocardial wall. After initial EGMs are recorded, the catheter may be repositioned, and fluorograms and EGMs may be recorded again. An electrical map may then be constructed from iterations of the process above.

According to an example, cardiac mapping may be generated based on detection of intracardiac electrical potential fields. A non-contact technique to simultaneously acquire a large amount of cardiac electrical information may be implemented. For example, a catheter having a distal end portion may be provided with a series of sensor electrodes distributed over its surface and connected to insulated electrical conductors for connection to signal sensing and processing means. The size and shape of the end portion may be such that the electrodes are spaced substantially away from the wall of the cardiac chamber. Intracardiac potential fields may be detected during a single cardiac beat. According to an example, the sensor electrodes may be distributed on a series of circumferences lying in planes spaced from each other. These planes may be perpendicular to the major axis of the end portion of the catheter. At least two additional electrodes may be provided adjacent at the ends of the major axis of the end portion. As a more specific example, the catheter may include four circumferences with eight electrodes spaced equiangularly on each circumference. Accordingly, in this specific implementation, the catheter may include at least 34 electrodes (32 circumferential and 2 end electrodes).

According to another example, an electrophysiological cardiac mapping system and technique based on a non-contact and non-expanded multi-electrode catheter may be implemented. EGMs may be obtained with catheters having multiple electrodes (e.g., between 42 to 122 electrodes). According to this implementation, knowledge of the relative geometry of the probe and the endocardium may be obtained such as by an independent imaging modality such as transesophageal echocardiography. After the independent imaging, non-contact electrodes may be used to measure cardiac surface potentials and construct maps therefrom. This technique may include the following steps (after the independent imaging step): (a) measuring electrical potentials with a plurality of electrodes disposed on a probe positioned in the heart 120; (b) determining the geometric relationship of the probe surface and the endocardial surface; (c) generating a matrix of coefficients representing the geometric relationship of the probe surface and the endocardial surface; and (d) determining endocardial potentials based on the electrode potentials and the matrix of coefficients.

According to another example, a technique and apparatus for mapping the electrical potential distribution of a heart chamber may be implemented. An intra-cardiac multi-electrode mapping catheter assembly may be inserted into a patient's heart 120. The mapping catheter assembly may include a multi-electrode array with an integral reference electrode, or, preferably, a companion reference catheter. The electrodes may be deployed in the form of a substantially spherical array. The electrode array may be spatially referenced to a point on the endocardial surface by the reference electrode or by the reference catheter which is brought into contact with the endocardial surface. The preferred electrode array catheter may carry a number of individual electrode sites (e.g., at least 24). Additionally, this example technique may be implemented with knowledge of the location of each of the electrode sites on the array, as well as knowledge of the cardiac geometry. These locations are preferably determined by a technique of impedance plethysmography.

According to another example, a heart mapping catheter assembly may include an electrode array defining a number of electrode sites. The mapping catheter assembly may also include a lumen to accept a reference catheter having a distal tip electrode assembly which may be used to probe the heart wall. The mapping catheter may include a braid of insulated wires (e.g., having 24 to 64 wires in the braid), and each of the wires may be used to form electrode sites. The catheter may be readily positionable in a heart 120 to be used to acquire electrical activity information from a first set of non-contact electrode sites and/or a second set of in-contact electrode sites.

According to another example, another catheter for mapping electrophysiological activity within the heart may be implemented. The catheter body may include a distal tip which is adapted for delivery of a stimulating pulse for pacing the heart or an ablative electrode for ablating tissue in contact with the tip. The catheter may further include at least one pair of orthogonal electrodes to generate a difference signal indicative of the local cardiac electrical activity adjacent the orthogonal electrodes.

According to another example, a process for measuring electrophysiologic data in a heart chamber may be implemented. The method may include, in part, positioning a set of active and passive electrodes into the heart 120, supplying current to the active electrodes, thereby generating an electric field in the heart chamber, and measuring the electric field at the passive electrode sites. The passive electrodes are contained in an array positioned on an inflatable balloon of a balloon catheter. In preferred embodiments, the array is said to have from 60 to 64 electrodes.

According to another example, cardiac mapping may be implemented using one or more ultrasound transducers. The ultrasound transducers may be inserted into a patient's heart 120 and may collect a plurality of ultrasound slices (e.g., two dimensional or three-dimensional slices) at various locations and orientations within the heart 120. The location and orientation of a given ultrasound transducer may be known and the collected ultrasound slices may be stored such that they can be displayed at a later time. One or more ultrasound slices corresponding to the position of a probe (e.g., a treatment catheter) at the later time may be displayed and the probe may be overlaid onto the one or more ultrasound slices.

According to other examples, body patches and/or body surface electrodes may be positioned on or proximate to a patient's body. A catheter with one or more electrodes may be positioned within the patient's body (e.g., within the patient's heart 120) and the position of the catheter may be determined by a system based on signals transmitted and received between the one or more electrodes of the catheter and the body patches and/or body surface electrodes. Additionally, the catheter electrodes may sense biometric data (e.g., LAT values) from within the body of the patient (e.g., within the heart 120). The biometric data may be associated with the determined position of the catheter such that a rendering of the patient's body part (e.g., heart 120) may be displayed and may show the biometric data overlaid on a shape of the body.

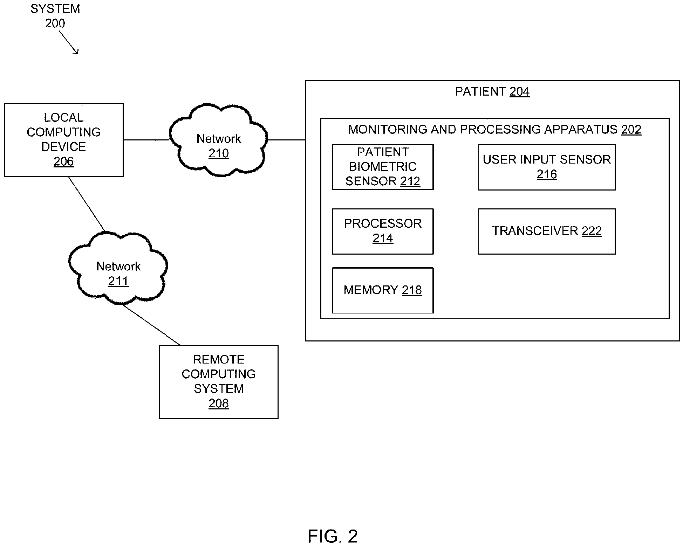

Turning now to FIG. 2, a block diagram of an example system 200 for remotely monitoring and communicating biometric data (i.e., patient biometrics, patient data, or patient biometric data) is illustrated. In the example illustrated in FIG. 2, the system 200 includes a monitoring and processing apparatus 202 (i.e., a patient data monitoring and processing apparatus) associated with a patient 204, a local computing device 206, a remote computing system 208, a first network 210, and a second network 211. In accordance with one or more embodiments, the monitoring and processing apparatus 202 may be an example of the catheter 105 of FIG. 1, the patient 204 may be an example of the patient 125 of FIG. 1, and the local computing device 206 may be an example of the console 160 of FIG. 1.

The monitoring and processing apparatus 202 includes a patient biometric sensor 212, a processor 214, a user input (UI) sensor 216, a memory 218, and a transmitter-receiver (i.e., transceiver) 222. In operation, the monitoring and processing apparatus 202 acquires biometric data of the patient 204 (e.g., electrical signals, blood pressure, temperature, blood glucose level or other biometric data) and/or receives at least a portion of the biometric data representing any acquired patient biometrics and additional information associated with any acquired patient biometrics from the one or more other patient biometric monitoring and processing apparatuses. The additional information may be, for example, diagnosis information and/or additional information obtained from an additional device such as a wearable device.

The monitoring and processing apparatus 202 may employ the autoencoder described herein to process data, including the acquired biometric data as well as any biometric data received from the one or more other patient biometric monitoring and processing apparatuses. For example, when processing data in this regard, the autoencoder may include a neural network that is used to learn latent representations (or data codings) in an unsupervised manner from the biometric data. Further, the autoencoder may learn to detect specific data by training the neural network to ignore signal interference, signal artifacts, and signal noise by considering clean datasets, without being pre-programmed with specific rules.

The monitoring and processing apparatus 202 may continually or periodically monitor, store, process, and communicate, via network 210, any number of various patient biometrics (e.g., the acquired biometric data). As described herein, examples of patient biometrics include electrical signals (e.g., ECG signals and brain biometrics), blood pressure data, blood glucose data, and temperature data. The patient biometrics may be monitored and communicated for treatment across any number of various diseases, such as cardiovascular diseases (e.g., arrhythmias, cardiomyopathy, and coronary artery disease) and autoimmune diseases (e.g., type I and type II diabetes).

The patient biometric sensor 212 may include, for example, one or more transducers configured to convert one or more environmental conditions into an electrical signal, such that different types of biometric data are acquired. For example, the patient biometric sensor 212 may include one or more of an electrode configured to acquire electrical signals (e.g., heart signals, brain signals, or other bioelectrical signals), a temperature sensor (e.g., thermocouple), a blood pressure sensor, a blood glucose sensor, a blood oxygen sensor, a pH sensor, an accelerometer, and a microphone.

As described in more detail herein, the monitoring and processing apparatus 202 may be an ECG monitor for monitoring ECG signals of a heart (e.g., the heart 120 of FIG. 1). In this regard, the patient biometric sensor 212 of the ECG monitor may include one or more electrodes (e.g., electrodes of the catheter 105 of FIG. 1) for acquiring ECG signals. The ECG signals may be used for treatment of various cardiovascular diseases.

In another example, the monitoring and processing apparatus 202 may be a continuous glucose monitor (CGM) for continuously monitoring blood glucose levels of a patient on a continual basis for treatment of various diseases, such as type I and type II diabetes. In this regard, the patient biometric sensor 212 of the CGM may include a subcutaneously disposed electrode (e.g., electrodes of the catheter 105 of FIG. 1), which may monitor blood glucose levels from interstitial fluid of the patient. The CGM may be, for example, a component of a closed-loop system in which the blood glucose data is sent to an insulin pump for calculated delivery of insulin without user intervention.

The processor 214 may be configured to receive, process, and manage, biometric data acquired by the patient biometric sensor 212, and communicate the biometric data to the memory 218 for storage and/or across the network 210 via the transceiver 222. Data from one or more other monitoring and processing apparatus 202 may also be received by the processor 214 through the transceiver 222, as described in more detail herein. Also, as described in more detail herein, the processor 214 may be configured to respond selectively to different tapping patterns (e.g., a single tap or a double tap) received from the UI sensor 216 (e.g., a capacitive sensor therein), such that different tasks of a patch (e.g., acquisition, storing, or transmission of data) may be activated based on the detected pattern. In some embodiments, the processor 214 can generate audible feedback with respect to detecting a gesture.

The UI sensor 216 may include, for example, a piezoelectric sensor or a capacitive sensor configured to receive a user input, such as a tapping or touching. For example, UI sensor 216 may be controlled to implement a capacitive coupling, in response to tapping or touching a surface of the monitoring and processing apparatus 202 by the patient 204. Gesture recognition may be implemented via any one of various capacitive types, such as resistive capacitive, surface capacitive, projected capacitive, surface acoustic wave, piezoelectric and infra-red touching. Capacitive sensors may be disposed at a small area or over a length of the surface, such that the tapping or touching of the surface activates the monitoring device.

The memory 218 is any non-transitory tangible media, such as magnetic, optical, or electronic memory (e.g., any suitable volatile and/or non-volatile memory, such as random-access memory or a hard disk drive). According to one or more embodiments, the memory 218 may store processor executable code, software, or instructions of a training algorithm and of the autoencoder.

The transceiver 222 may include a separate transmitter and a separate receiver. Alternatively, the transceiver 222 may include a transmitter and receiver integrated into a single device.

According to an embodiment, the monitoring and processing apparatus 202 may be an apparatus that is internal to a body of the patient 204 (e.g., subcutaneously implantable). The monitoring and processing apparatus 202 may be inserted into the patient 204 via any applicable manner including orally injecting, surgical insertion via a vein or artery, an endoscopic procedure, or a laparoscopic procedure.

According to an embodiment, the monitoring and processing apparatus 202 may be an apparatus that is external to the patient 204. For example, as described in more detail herein, the monitoring and processing apparatus 202 may include an attachable patch (e.g., that attaches to a patient's skin). The monitoring and processing apparatus 202 may also include a catheter with one or more electrodes, a probe, a blood pressure cuff, a weight scale, a bracelet or smart watch biometric tracker, a glucose monitor, a continuous positive airway pressure (CPAP) machine or virtually any device which may provide an input concerning the health or biometrics of the patient.

According to an embodiment, a monitoring and processing apparatus 202 may include both components that are internal to the patient and components that are external to the patient.

While a single monitoring and processing apparatus 202 is shown in FIG. 2, example systems may include a plurality of patient biometric monitoring and processing apparatuses. For instance, the monitoring and processing apparatus 202 may be in communication with one or more other patient biometric monitoring and processing apparatuses. Additionally, or alternatively, the one or more other patient biometric monitoring and processing apparatus may be in communication with the network 210 and other components of the system 200.

The local computing device 206 and/or the remote computing system 208, along with the monitoring and processing apparatus 202, may be any combination of software and/or hardware that individually or collectively store, execute, and implement the autoencoder and functions thereof. Further, the local computing device 206 and/or the remote computing system 208, along with the monitoring and processing apparatus 202, may be an electronic, computer framework including and/or employing any number and combination of computing device and networks utilizing various communication technologies, as described herein. The local computing device 206 and/or the remote computing system 208, along with the monitoring and processing apparatus 202, may be easily scalable, extensible, and modular, with the ability to change to different services or reconfigure some features independently of others.

According to an embodiment, the local computing device 206 and the remote computing system 208, along with the monitoring and processing apparatus 202, may include at least a processor and a memory, where the processor executes computer instructions with respect the autoencoder and the memory stores the instructions for execution by the processor.

The local computing device 206 of system 200 is in communication with the monitoring and processing apparatus 202 and may be configured to act as a gateway to the remote computing system 208 through the second network 211. The local computing device 206 may be, for example, a, smart phone, smartwatch, tablet or other portable smart device configured to communicate with other devices via network 211. Alternatively, the local computing device 206 may be a stationary or standalone device, such as a stationary base station including, for example, modem and/or router capability, a desktop or laptop computer using an executable program to communicate information between the processing apparatus 202 and the remote computing system 208 via the PC's radio module, or a USB dongle. Biometric data may be communicated between the local computing device 206 and the monitoring and processing apparatus 202 using a short-range wireless technology standard (e.g., Bluetooth, Wi-Fi, ZigBee, Z-wave and other short-range wireless standards) via the short-range wireless network 210, such as a local area network (LAN) (e.g., a personal area network (PAN)). In some embodiments, the local computing device 206 may also be configured to display the acquired patient electrical signals and information associated with the acquired patient electrical signals, as described in more detail herein.

In some embodiments, the remote computing system 208 may be configured to receive at least one of the monitored patient biometrics and information associated with the monitored patient via network 211, which is a long-range network. For example, if the local computing device 206 is a mobile phone, network 211 may be a wireless cellular network, and information may be communicated between the local computing device 206 and the remote computing system 208 via a wireless technology standard, such as any of the wireless technologies mentioned above. As described in more detail herein, the remote computing system 208 may be configured to provide (e.g., visually display and/or aurally provide) the at least one of the patient biometrics and the associated information to a medical professional, a physician, a healthcare professional, or the like.

In FIG. 2, the network 210 is an example of a short-range network (e.g., local area network (LAN), or personal area network (PAN)). Information may be sent, via short-range network 210, between the monitoring and processing apparatus 202 and the local computing device 206 using any one of various short-range wireless communication protocols, such as Bluetooth, Wi-Fi, Zigbee, Z-Wave, near field communications (NFC), ultraband, Zigbee, or infrared (IR).

The network 211 may be a wired network, a wireless network or include one or more wired and wireless networks, such as an intranet, a local area network (LAN), a wide area network (WAN), a metropolitan area network (MAN), a direct connection or series of connections, a cellular telephone network, or any other network or medium capable of facilitating communication between the local computing device 206 and the remote computing system 208. Information may be sent, via the network 211 using any one of various long-range wireless communication protocols (e.g., TCP/IP, HTTP, 3G, 4G/LTE, or 5G/New Radio). Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology. Additionally, several networks may work alone or in communication with each other to facilitate communication in the network 211. In some instances, the remote computing system 208 may be implemented as a physical server on the network 211. In other instances, the remote computing system 208 may be implemented as a virtual server a public cloud computing provider (e.g., Amazon Web Services (AWS) ^{®}) of the network 211.

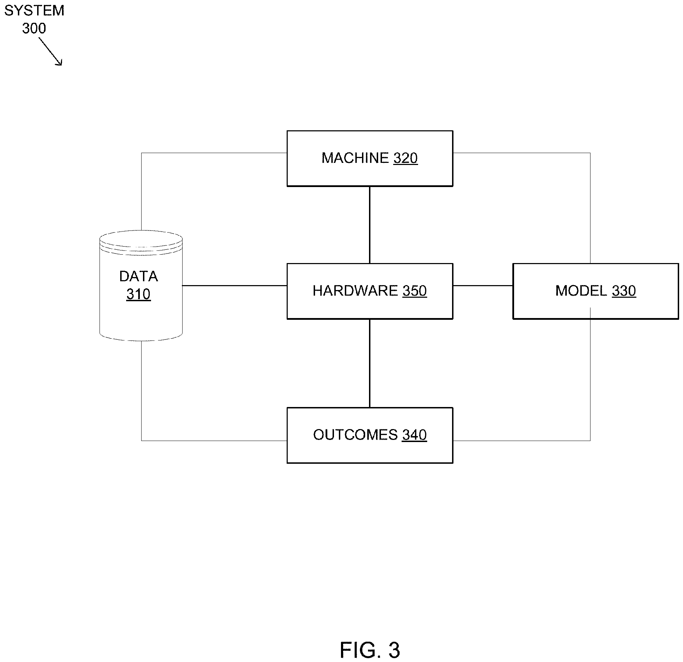

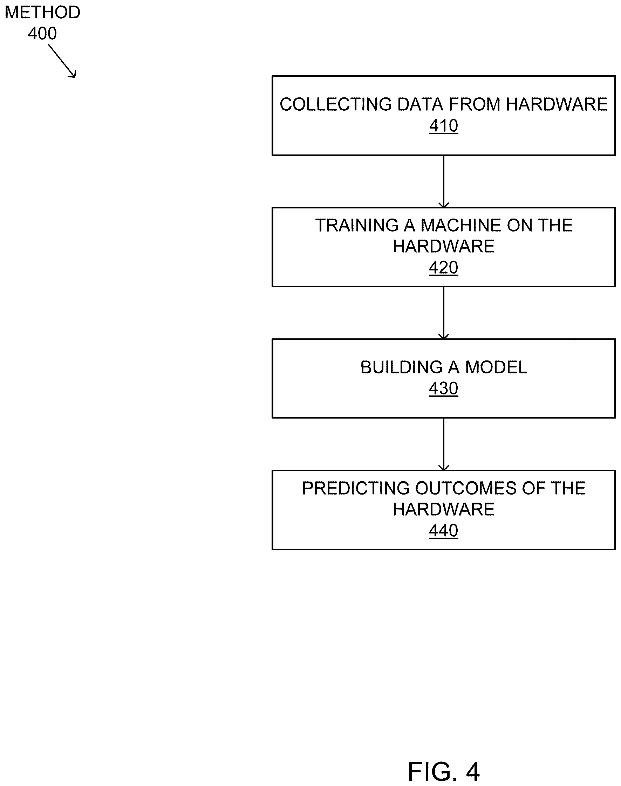

FIG. 3 illustrates an artificial intelligence system 300 according to one or more embodiments. The artificial intelligence system 300 may include data 310, a machine 320, a model 330, a plurality of outcomes 340, and underlying hardware 350. FIG. 4 illustrates a block diagram of a method 400 performed in the artificial intelligence system of FIG. 3. The description of FIGS. 3-4 is made with reference to FIG. 2 for ease of understanding.

In general, the artificial intelligence system 300 operates the method 400 by using the data 310 to train the machine 320 (e.g., the local computing device 206 of FIG. 2) while building the model 330 to enable the plurality of outcomes 340 (to be predicted). In such a configuration, the artificial intelligence system 300 may operate with respect to the hardware 350 (e.g., the monitoring and processing apparatus 202 of FIG. 2) to train the machine 320, build the model 330, and predict outcomes using algorithms. These algorithms may be used to solve the trained model 330 and predict outcomes 340 associated with the hardware 350. These algorithms may be divided generally into classification, regression, and clustering algorithms.

At block 410, the method 400 may include collecting the data 310 from the hardware 350. The machine 320 may operate as the controller or data collection associated with the hardware 350 and/or is associated therewith. The data 310 (e.g., biometric data, which may originate with the monitoring and processing apparatus 202 of FIG. 2) may be related to the hardware 350. For instance, the data 310 may be on-going data, or output data associated with the hardware 350. The data 310 may also include currently collected data, historical data, or other data from the hardware 350. For example, the data 310 may include measurements during a surgical procedure and may be associated with an outcome of the surgical procedure. For example, a temperature of a heart (e.g., of the patient 204) may be collected and correlated with an outcome of a heart procedure.

At block 420, the method 400 may include training the machine 320, such as with respect to the hardware 350. The training may include an analysis and correlation of the data 310 collected at block 410. For example, in the case of the heart, the data 310 of temperature and outcome may be trained to determine if a correlation or link exists between the temperature of the heart (e.g., of the patient 204) during the heart procedure and the outcome.

At block 430, the method 400 may include building the model 330 on the data 310 associated with the hardware 350. Building the model 330 may include physical hardware or software modeling, algorithmic modeling, and/or the like. This modeling may seek to represent the data 310 that has been collected and trained. According to an embodiment, the model 330 may be configured to model the operation of hardware 350 and model the data 310 collected from the hardware 350 to predict the outcome achieved by the hardware 350. In accordance with one or more embodiments, the model 330, with respect to the autoencoder, may separate between ventricular far field and atrial based activation and generate separate maps for atrial and ventricular activation.

At block 440, the method 400 may include predicting the plurality of outcomes 340 of the model 330 associated with the hardware 350. This prediction of the plurality of outcomes 340 may be based on the trained model 330. For example and to increase understanding of the disclosure, in the case of the heart, if the temperature during the procedure is between 36.5 degrees Celsius and 37.89 degrees Celsius (i.e., 97.7 degrees Fahrenheit and 100.2 degrees Fahrenheit) produces a positive result from the heart procedure, the outcome can be predicted in a given procedure based on the temperature of the heart during the heart procedure. Thus, using the outcome 340 that is predicted, the hardware 350 may be configured to provide a certain desired outcome 340 from the hardware 350.

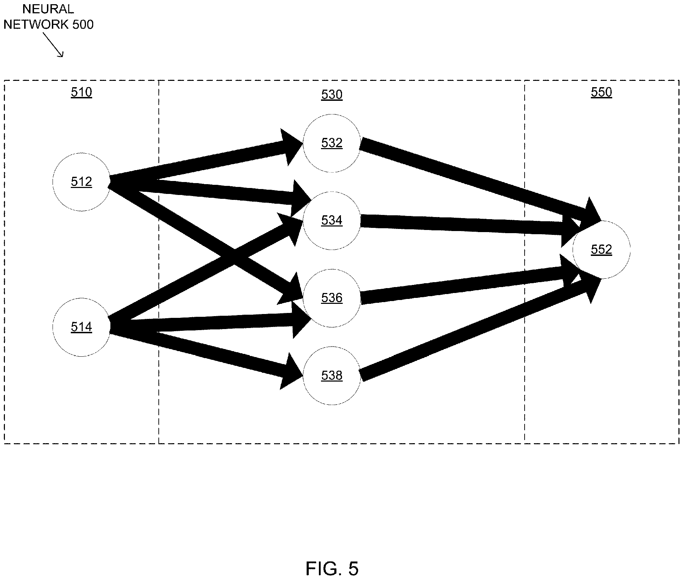

Turning now to FIG. 5, an example of a neural network 500 is illustrated according to one or more embodiments. The neural network 500 may operate as an implementation of the autoencoder. The neural network 500 may be implemented in hardware, such as the machine 320 (e.g., the local computing device 206 of FIG. 2) and/or the hardware 350 (e.g., the monitoring and processing apparatus 202 of FIG. 2). In general, a neural network is a network or circuit of neurons, or in a modern sense, an artificial neural network (ANN), composed of artificial neurons or nodes or cells.

For example, an ANN may involve a network of processing elements (artificial neurons) which may exhibit complex global behavior, determined by the connections between the processing elements and element parameters. These connections of the network or circuit of neurons may be modeled as weights. A positive weight may reflect an excitatory connection, while negative values may mean inhibitory connections. Inputs may be modified by a weight and summed using a linear combination. An activation function may control the amplitude of the output. For example, an acceptable range of output is usually between 0 and 1, or it could be -1 and 1.

In most cases, the ANN is an adaptive system that changes its structure based on external or internal information that flows through the network. In more practical terms, neural networks are non-linear statistical data modeling or decision-making tools that can be used to model complex relationships between inputs and outputs or to find patterns in data. Thus, ANNs may be used for predictive modeling and adaptive control applications, while being trained via a dataset. Self-learning resulting from experience may occur within ANNs, which may derive conclusions from a complex and seemingly unrelated set of information. The utility of artificial neural network models lies in the fact that they can be used to infer a function from observations and also to use it. Unsupervised neural networks can also be used to learn representations of the input that capture the salient characteristics of the input distribution, and more recently, deep learning algorithms, which can implicitly learn the distribution function of the observed data. Learning in neural networks is particularly useful in applications where the complexity of the data or task makes the design of such functions by hand impractical.

Neural networks may be used in different fields. The tasks to which ANNs are applied tend to fall within the following broad categories: function approximation, or regression analysis, including time series prediction and modeling; classification, including pattern and sequence recognition, novelty detection and sequential decision making, data processing, including filtering, clustering, blind signal separation and compression.

Application areas of ANNs may include nonlinear system identification and control (vehicle control, process control), game-playing and decision making (backgammon, chess, racing), pattern recognition (radar systems, face identification, object recognition), sequence recognition (gesture, speech, handwritten text recognition), medical diagnosis, financial applications, data mining (or knowledge discovery in databases, "KDD"), visualization and e-mail spam filtering. For example, it is possible to create a semantic profile of user's interests emerging from pictures trained for object recognition.

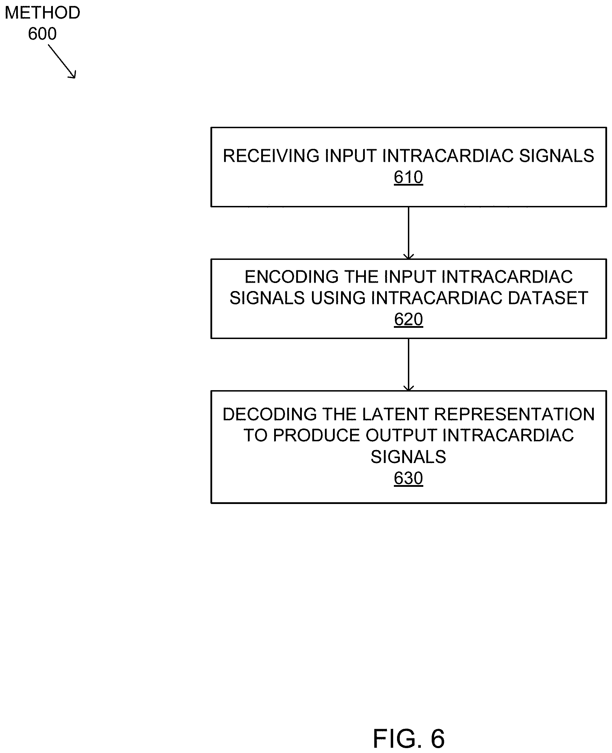

Turning now to FIG. 6, a block diagram of a method 600 is illustrated according to one or more embodiments. The method 600 depicts operations of the neural network 500 (e.g., an autoencoder). Returning to FIG. 5, in the neural network 500, an input layer 510 is represented by a plurality of inputs, such as 512 and 514. With respect to block 610 of FIG. 6, the input layer 510 may receive the plurality of inputs (e.g., input intracardiac signals) as an initial operation. The plurality of inputs may be ultrasound signals, radio signals, audio signals, or a two-dimensional picture. More particularly, the plurality of inputs may be represented as input data (X), which is raw data recorded from an atria. Desired information may lie in high frequency zones of the heart (e.g., the atrium), and the autoencoder provides a better construction of the input intracardiac signals. In accordance with one or more embodiments, the plurality of inputs can be a combination of intracardiac and body surface ECG (to remove far field noise from intracardiac signals).

At block 620 of FIG. 6, the neural network 500 may encode the input intracardiac signals utilizing an intracardiac dataset to produce a latent representation. The latent representation may include one or more intermediary images derived from the input intracardiac signals. According to one or more embodiments, the latent representation may be generated by an element-wise activation function (e.g., a sigmoid function or a rectified linear unit) of the autoencoder that applies a weight matrix to the input intracardiac signals and adds a bias vector to the result. Weights and biases of the weight matrix and the bias vector may be initialized randomly, and then updated iteratively during training.

The intracardiac dataset may be a training dataset or clean data that includes predetermined and approved signals that are free from interferences, artifacts, and noise (i.e., an example of clean). In an embodiment, an expert medical professional, a physician, or the like may review, edit to remove signal interferences, signal artifacts, and signal noise, and approve each electrical signal of the intracardiac dataset. In an embodiment, the intracardiac data may have a number of electrical signals on the order of thousands or greater, where a signal morphology of each electrical signal is examined using template matching and blanking. For example, with an intracardiac dataset (e.g., a database of "clean version" of intracardiac ECG signals), then denoising of any IC-ECG artifact may be performed. Given the volume of electrical signals and the complexity of reviewing, editing, and approving, creation of the intracardiac dataset may be considered a data training portion of the multi-step data manipulation by the autoencoder.

As shown in FIG. 5, the inputs 512 and 514 may be provided to a hidden layer 530 depicted as including nodes 532, 534, 536, and 538 (e.g., the latent representation or data coding). This encoding provides a dimensionality reduction of the input intracardiac signals. Dimensionality reduction is a process of reducing the number of random variables (of the plurality of inputs) under consideration by obtaining a set of principal variables. For instance, dimensionality reduction can be a feature extraction that transforms data (e.g., the plurality of inputs) from a high-dimensional space (e.g., more than 10 dimensions) to a lower-dimensional space (e.g., 2-3 dimensions). The technical effects and benefits of dimensionality reduction include reducing time and storage space requirements for the data, improving visualization of the data, and improving parameter interpretation for machine learning. This data transformation may be linear or nonlinear. The operations of receiving (block 610) and encoding (block 620) may be considered a data preparation portion of the multi-step data manipulation by the autoencoder.

According to an embodiment, the data preparation may further include intracardiac-electrocardiograms (IC-ECG) data collection of the atria (upper chambers through which blood enters the ventricles of the heart) with simultaneous recordings from the ventricle (the two lower chambers of the heart).

At block 630 of FIG. 6, the neural network 500 may decode the latent representation to produce output intracardiac signals. The output intracardiac signals may be a ventricular far field estimation in the case of IC-ECGs. As shown in FIG. 5, the nodes 532, 534, 536, and 538 may be combined to produce an output 552 in an output layer 550, where the output layer 550 may reconstruct the inputs 512 and 514 on a reduced dimension but without the signal interferences, signal artifacts, and signal noise. The neural network 500 may perform the processing via the hidden layer 530 of the nodes 532, 534, 536, and 538 to exhibit complex global behavior, determined by the connections between the processing elements and element parameters. The target data for the output layer 550 may include target data type one ventricular activity (Y1) and includes target data type two input data after far field reduction (Y2). Far field may cause problems with respect to generating and navigating 3D maps (e.g., ventricular far field may interfere with atrial activation). Thus, a technical effect and benefit of the autoencoder employing the neural network 500 includes improving the accuracy of 3D maps due to artifact (with respect to the far field) removal.

In accordance with one or more embodiments, a model of the autoencoder employing the neural network 500 may separate between ventricular far field and atrial based activation and generate separate maps for atrial and ventricular activation.

In accordance with an embodiment, the autoencoder may be a denoising autoencoder to find mapping functions (f, g) such that f(X) = Y1 and g(X) = Y2, as further described herein. In this regard, a task of an autoencoder may be to learn a mapping from X to X, through some dimensional reduction of the input X (e.g., build two neural networks (F, G) such that h =F(X) and X = G(h). The dimension of h is smaller than the dimension of X. In a denoising autoencoder, while the architecture is similar, the denoising autoencoder learns a mapping from X to Y, where Y is a denoised version of X.

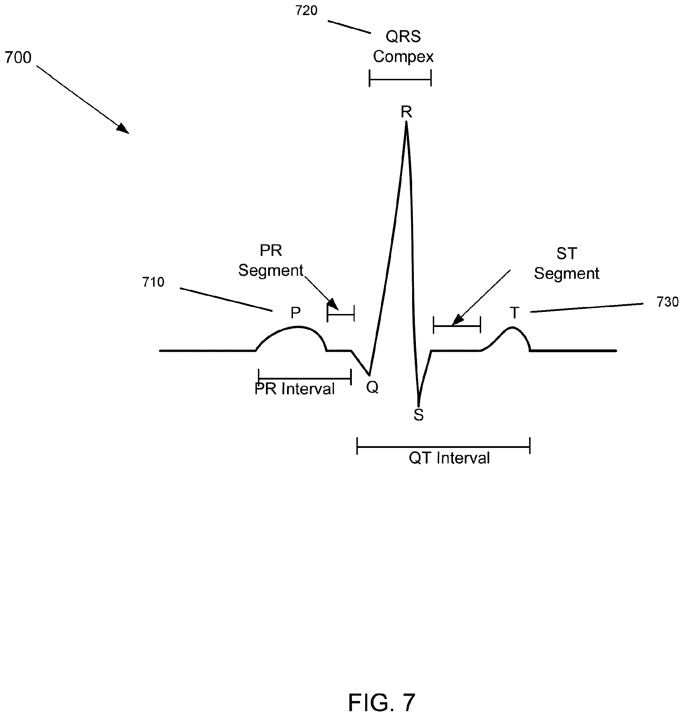

Turning to FIG. 7, a graphical depiction of a signal 700 is illustrated according to one or more embodiments. As shown by the signal 700, an ECG signal contains a P wave 710 (due to atrial depolarization), a QRS complex 720 (due to atrial repolarization and ventricular depolarization) and a T wave 730 (due to ventricular repolarization). An ECG signal is generated by contraction (depolarization) and relaxation (repolarization) of atrial and ventricular muscles of the heart. To record an ECG signal, electrodes may be placed at specific positions on the human body or can be positioned within a human body via a catheter. Artifacts (e.g., noise) are the unwanted signals that are merged with electrical signals such as ECG signals, and sometimes create obstacles for the diagnosis and/or treatment of a cardiac condition. Artifacts in electrical signals can be baseline wander, powerline interference, EMG noise, power line noise, etc. That is, examples of artifacts include, but are not limited to, power noise (e.g., electrostatic and electromagnetic coupling between circuity and 50 or 60 Hz power lines), Fluro noise (e.g., fluorescent lights), contact noise (e.g., collision between catheter electrodes), and deflection noise (e.g., discharges of static electricity during catheter deflection).

Baseline wander or baseline drift may occur where the base axis (x-axis) of a signal appears to 'wander' or move up and down rather than be straight. This may cause the entire signal to shift from its normal base. In ECG signals, the baseline wander may be caused due to improper electrode contact (e.g., electrode-skin impedance), patient movement, and cyclical movement (e.g., respiration).

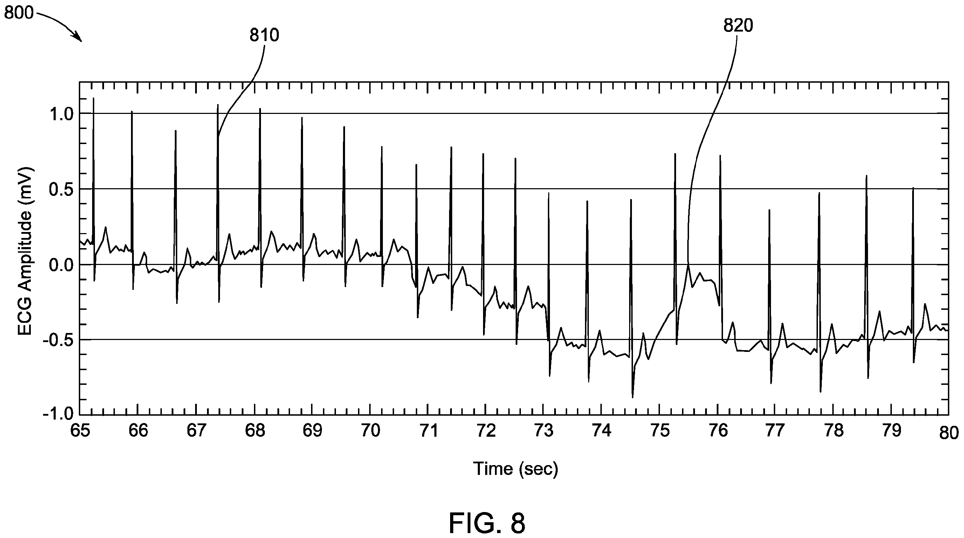

FIG. 8 illustrates a graphical depiction of a signal 810 is illustrated in a plot 800 according to one or more embodiments. In this regard, the signal 800 is a typical ECG signal affected by baseline wander 820. The frequency content of the baseline wander is in the range of 0.5 Hz. Increased movement of the body during exercise or stress test increases the frequency content of baseline wander. According to implementations, given that the baseline signal is a low frequency signal, a Finite Impulse Response (FIR) high-pass zero phase forward-backward filtering with a cutoff frequency of 0.5 Hz to estimate and remove the baseline wander 820 in the ECG signal 810 can be used.

Electromagnetic fields caused by a powerline represent a common noise source in electrical signals such as ECGs, as well as to any other bioelectrical signal recorded from a patient's body. Such noise is characterized by, for example, 50 or 60 Hz sinusoidal interference, possibly accompanied by a number of harmonics. Such narrowband noise renders the analysis and interpretation of the ECG more difficult, since the delineation of low-amplitude waveforms becomes unreliable and spurious waveforms may be introduced. It may be necessary to remove powerline interference from ECG signals as it superimposes the low frequency ECG waves like P wave 710 and T wave 730.

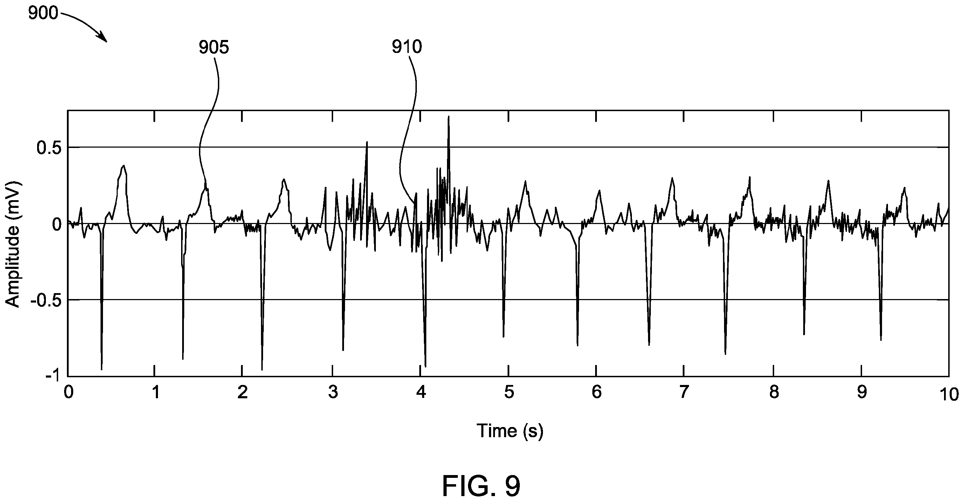

The presence of muscle noise can interfere with in many electrical signal applications such as ECG applications, as low amplitude waveforms can become obscured. Muscle noise is, in contrast to baseline wander 820 and 50/60 Hz interference, not removed by narrowband filtering, but presents a different filtering problem as the spectral content of muscle activity considerably overlaps that of the PQRST complex 720. As an ECG signal 810 is a repetitive signal, techniques can be used to reduce muscle noise in a manner similar to the processing of evoked potentials. FIG. 9 illustrates a graphical depiction 900 of a signal 905 illustrated according to one or more embodiments. In this regard, the signal 905 is an ECG signal interfered by an EMG noise 910.

Instruments for measuring electrical signals such as ECG signals often detect electrical interference corresponding to a line, or mains, frequency. Line frequencies in most countries, though nominally set at 50 Hz or 60 Hz, may vary by several percent from these nominal values.

Various techniques for removing electrical interference from electrical signals can be implemented. Several of these techniques use of one or more low-pass or notch filters. For example, a system for variable filtering of noise in ECG signals may be implemented. The system may have a plurality of low pass filters including one filter with a, for example, 3 dB point at approximately 50 Hz and, for example, a second low pass filter with a 3 dB point at approximately 5 Hz.

According to another example, a system for rejecting a line frequency component of an electrical signal may be implemented by passing the signal through two serially linked notch filters. A system with a notch filter that may have either or both low-pass and high-pass coefficients for removing line frequency components from an ECG signal may be implemented. The system may also support removal of burst noise and calculate a heart rate from the notch filter output.

According to another example, a system with several units for removing interference may be implemented. The units may include a mean value unit to generate an average signal over several cardiac cycles, a subtracting unit to subtract the average signal from the input signal to generate a residual signal, a filter unit to provide a filtered signal from the residual signal, and/or an addition unit to add the filtered signal to the average signal.

According to another example, an analog-to-digital (A/D) converter may provide noise rejection by synchronizing a clock of the converter with a phase locked loop set to the line frequency.

Additionally, biometric (e.g., biopotential) patient monitors may use surface electrodes to make measurements of bioelectric potentials such as ECG or electroencephalogram (EEG). The fidelity of these measurements is limited by the effectiveness of the connection of the electrode to the patient. The resistance of the electrode system to the flow of electric currents, known as the electric impedance, characterizes the effectiveness of the connection. Typically, the higher the impedance, the lower the fidelity of the measurement. Several mechanisms may contribute to lower fidelity.

Signals from electrodes with high impedances are subject to thermal noise (or so-called Johnson noise), voltages that increase with the square root of the impedance value. In addition, biopotential electrodes tend to have voltage noises in excess of that predicted by Johnson. Also, amplifier systems making measurements from biopotential electrodes can have degraded performance at higher electrode impedances. The impairments are characterized by poor common mode rejection, which tends to increase the contamination of the bioelectric signal by noise sources such as patient motion and electronic equipment that may be in use on or around the patient. These noise sources are particularly prevalent in the operating theatre and may include equipment such as electrosurgical units (ESU), cardiopulmonary bypass pumps (CPB), electric motor-driven surgical saws, lasers and other sources.

During a cardiac procedure, it is often desirable to measure electrode impedances continuously in real time while a patient is being monitored. To do this, a very small electric current is typically injected through the electrodes and the resulting voltage measured, thereby establishing the impedance using Ohm's law. This current may be injected using DC or AC sources. It is often not possible to separate voltage due to the electrode impedance from voltage artifacts arising from interference. Interference tends to increase the measured voltage and thus the apparent measured impedance, causing the biopotential measurement system to falsely detect higher impedances than are actually present. Often such monitoring systems have maximum impedance threshold limits that may be programmed to prevent their operation when they detect impedances in excess of these limits. This is particularly true of systems that make measurements of very small voltages, such as the EEG. Such systems require very low electrode impedances.

The use of high resolution intra-cardiac electrograms (EGMs) may guide cardiac ablation procedures. Cardiac ablation, among others, may be used to treat ventricular tachycardia (VT) where fast and irregular heartbeat results from complex electro-physiological (EP) circuits and re-entry in one of the ventricles. Thus, catheter ablation aim is to target the origin of VT. Mapping of VT circuits and identification of their source are crucial for the success of VT ablation. A major challenge in interpreting intra-cardiac EGMs in the presence of VT is that EGM signals can have a complex morphology, making it difficult to extract the local activation time (LAT) with sufficiently high spatial resolution. This in turn makes it difficult to precisely map the complex circuits in the ventricle, which is critical for locating relevant ablation targets.

Unipolar EGM signals typically have much lower signal to noise ratio compared to bipolar signals, and thus bipolar signals are currently the main tool for extracting LATs. However, unipolar signals potentially offer better spatial and temporal resolution, which may significantly improve the mapping of the VT circuits. Thus, advanced digital signal processing (DSP) methods may be applied to extract accurate LATs from noisy unipolar signals. An advanced digital signal processing method or system aims to reduce or diminish noise from a signal and may include a variety of digital filters. A linear smoothing filter, for example, a low-pass filter or high-pass filter, or any other smoothing operator, that may be convolved with the signal, may be used to reduce or diminish noise. A non-linear filter, for example a median filter for denoising, may be used to reduce or diminish noise. A wavelet transform, that may accomplish both noise reduction and feature preservation may be used. Statistical denoising methods may be used, which may use the environment or neighbor signals or any other patterns, to reduce unwanted components in the signal.

A bipolar signal is from two adjacent unipolar electrodes. A unipolar signal is from a unipolar electrode and a reference electrode and is a combination of far field and near field contributions. A main source of noise in a unipolar signal is far field signals resulting from voltage depolarization of remote tissue. Due to a large distance between a unipolar electrode and a reference electrode, the far field signals are often not fully accounted for and therefore not fully removed from the signal as compared to bipolar signals. Since the two unipolar signals that form a unipolar pair have very similar far fields, the difference between them leads to almost zero, except where there is a local activity at each of the unipolar signals. This local activity is referred to as the near field signal and can be indicated as small spikes over the bipolar signals.

In cases where the unipolar electrode is located under scarred tissue that does not create electric activity, the near field may have a lower amplitude than the far field as compared to situations arising from healthy tissue. This makes it particularly difficult to apply classical DSP methods to separate far field signals from near field signals. In this case, the near field may be very low and negligible. Therefore, the bipolar signal may not have any activations and may be effectively zero. This type of unipolar can be represented as pure far field signals, since there is no local activity evident. In this case, the bipolar signal may appear flat and the two unipolar signals may be almost the same. These types of signals may be obtained by placing the catheter in a position without contacting with the heart muscle or as body surface ECG signals, which are basically far field signals. These types of unipolar signals may be a training data set to make a neural network learn this type of activity, and to be able to distinguish the far field component from the mixed unipolar signal.

While in the current context, the far field contribution is considered as noise to be removed, in other contexts the far field contribution itself may contain useful information. This may provide additional motivation for the separation of the two contributions.

Deep Learning (DL) based on deep neural networks (DNNs) has emerged as a disruptive technology in the application of computer algorithms to various fields, such as computer vision and DSP. DL allows complex patterns and data to be extracted from signals and images, often in cases where such extraction was previously impossible, or possible only with time-consuming manual analysis. Thus, DL is particularly attractive for application to intracardiac EGMs, where reducing procedure time and increasing clinical success rate are key goals.

Machine learning (ML) is a group of algorithms and statistical models for data parsing which are used to perform a specific task. DL is a subset of machine learning algorithms, which set model parameters during a training process to allow an accurate prediction of a desired output for an unseen data. ML and DL techniques allow an analysis of highly complex spatio-temporal information which classical algorithms struggle to analyze. While machine learning is usually based on feature extraction using a list of heuristics regarding the data, DL is based on learning from examples and typically does not require the extraction of features from the data. A major difference between DL and traditional ML is the need of large amounts of data for the training process. Given a sufficient amount of data, the performance of DL based algorithms is typically superior to traditional ML algorithms.

Accordingly, DL are useful tools to decompose near field and far field components in ECG signals, and specifically VT signals. This will allow activation detection only on near field activity. This is useful since, while mapping ventricle activity, far field can be strong and mask the near field activity, thus misleading the annotation mechanism. It is also useful in a case of atrial fibrillation (AFIB) since the ventricles signal is strong it may be mis-annotated as atrial activity.

It is therefore desirable for DL methods to shorten the time of an entire clinical procedure by providing the medical personnel (e.g., cardiologists and electrophysiologists) with insight that currently is only available through manual data analysis by trained clinicians and to identify deep data patterns that currently cannot be identified manually or using classical algorithms (e.g., DSP and computer vision), and thus allow identification of ablation targets in more complex cases which are currently untreatable.

DL training may be unsupervised. That is, while a large body of prerecorded unipolar EGM signals exists, and additional signals may be collected if needed, a major challenge in applying DL for the removal of far field noise is the lack of ground truth data with which to train the DL model. Any far and near field signal decomposition is an assessment and cannot necessarily be compared to the real far and near field signals at the specific electrode. Therefore, a DL approach may be unsupervised rather than supervised. The body surface ECG may be used with distant electrodes as a ground truth for the far field component.

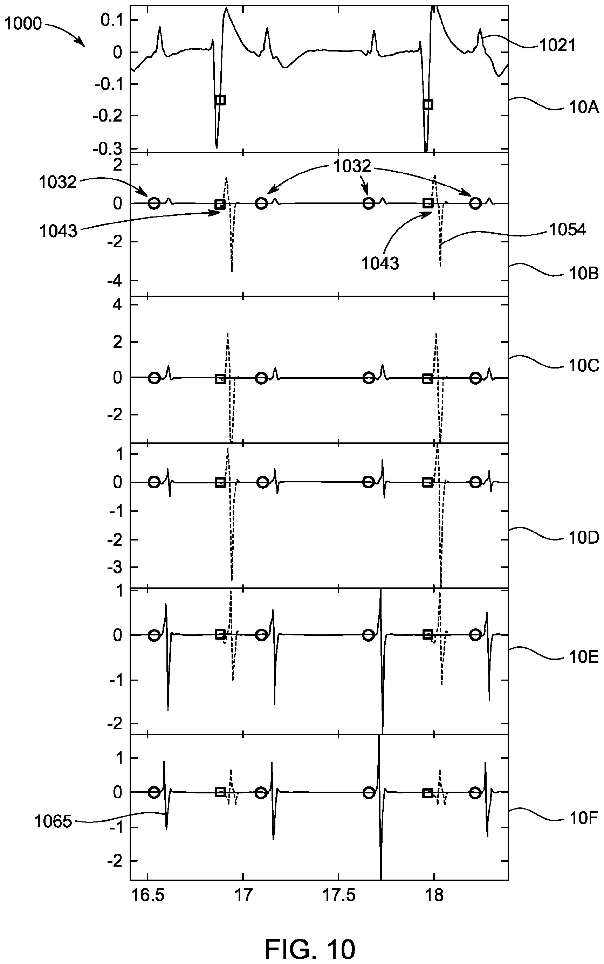

FIG. 10 illustrates a graphical depiction of a signal progression 1000 (10A, 10B, 10C, 10D, 10E, and 10F) of far field removal according to one or more embodiments. The signals in figures 10A-10E are intracardiac (IC) ECG signals recorded from different locations along a coronary sinus (CS). In FIG. 10A, a signal 1021 represents a body surface IC ECG signal. A demarcation 1032 represents a local activation time (LAT). Demarcation 1032 is also present in FIG. 10C, 10D, 10E, and 10F. A demarcation 1043 represents QRS location. Demarcation 1043 is also present in FIG. 10A, FIG. 10C, 10D, 10E, and 10F. In FIG. 10, the X-axis represents time, while the Y-axis represents mV.

As shown in FIG 10, 1054 represents a far field component of the IC)ECG signal. FIG. 10C, 10D, 10E, and 10F show a progression of signal 1054 with an increasing amount of far field removal with signal 1065 representing the IC ECG signal after far field removal. Far field removal may be achieved by, for example, be creating a blanking period, for example the IC ECG signal 1065 may be zero during the far field period.

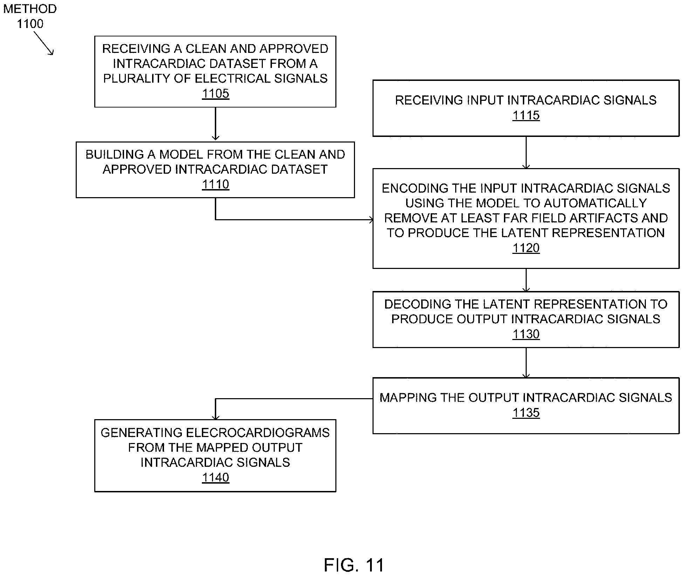

FIG. 11 illustrates a block diagram of a method 1100 according to one or more embodiments. In accordance with an embodiment, the method 1100 may be implemented by a denoising autoencoder. Any combination of software and/or hardware (e.g., the local computing device 206 and the remote computing system 208, along with the monitoring and processing apparatus 202) may individually or collectively store, execute, and implement the denoising autoencoder and functions thereof. The denoising autoencoder may train an autoencoder to reconstruct an input from a corrupted version of itself to force a hidden layer (e.g., the hidden layer 530 of FIG. 5) to discover more robust features (i.e., useful features that will constitute better higher level representations of the input) and prevent it from learning a particularly identity (i.e., always returning to a same value). In this regard, the denoising autoencoder may encode the input (e.g., to preserve information about the input) and may reverse the effect of a corruption process stochastically applied to the input of an autoencoder.

According to one or more embodiments, the denoising autoencoder may implement a long short-term memory neural network architecture, a convolutional neural network architecture, or other the like. The architecture of the denoising autoencoder may be configurable with respect to a number of layers, a number of connections (e.g., encoder/decoder connections), a regularization technique (e.g., dropout or BN); and an optimization feature.

The long short-term memory neural network architecture may include feedback connections and may process single data points (e.g., such as images), along with entire sequences of data (e.g., such as speech or video). A unit of the long short-term memory neural network architecture may be composed of a cell, an input gate, an output gate, and a forget gate, where the cell remembers values over arbitrary time intervals and the gates regulate a flow of information into and out of the cell.

The convolutional neural network architecture may be a shared-weight architecture with translation invariance characteristics where each neuron in one layer is connected to all neurons in the next layer. The regularization technique of the convolutional neural network architecture may take advantage of the hierarchical pattern in data and assemble more complex patterns using smaller and simpler patterns. If the denoising autoencoder implements the convolutional neural network architecture, other configurable aspects of the architecture may include a number of filters at each stage, kernel size, a number of kernels per layer.

The method 1100 begins at block 1105, where the denoising autoencoder may receive a "clean and approved" intracardiac dataset from a plurality of electrical signals. As indicated herein, an expert medical professional, a physician, or the like may review and edit the dataset to remove signal interferences, signal artifacts, and signal noise, and approve each electrical signal of the intracardiac dataset. At block 1110, the denoising autoencoder may build a model (e.g., the model 330 of FIG. 3) from the clean and approved intracardiac dataset.

At block 1115, the denoising autoencoder may receive input intracardiac signals, which include at least far field artifacts. The input intracardiac signals may be recorded by one or more monitoring and processing apparatuses (e.g., a penta-ray catheter with twenty electrodes, a basket catheter with sixty-four electrodes, a plurality of body surface leads, etc.) Far field may cause problems regarding generating and navigating 3D maps (i.e., ventricular far field may interfere with atrial activation).

At block 1120, the denoising autoencoder may encode the input intracardiac signals using the model (from block 1110). This encoding provides a dimensionality reduction of the input intracardiac signals, according to how the model dictates the reduction, that remove at least far field artifacts. The result of the encoding is a production of a latent representation. At block 1130, the denoising autoencoder may decode the latent representation to produce output intracardiac signals.

At block 1135, the denoising autoencoder may map the output intracardiac signals. For example, the denoising autoencoder, utilizing its underlying architecture, finds mapping functions (f, g), such that f(X) = Y1 and g(X) = Y2.

At block 1140, ECGs may be generated from the mapped output intracardiac signals. The ECGs may be generated by a computing device that is executing the denoising autoencoder or by another device. The ECGs, which are improved because the signal interference, the signal noise, and the signal artifacts are removed, may then be displayed to a medical professional. The improved ECGs may dramatically reduce the time spent on a cardiac case.

As indicated herein, during intracardiac-electrogram mapping, the mapping catheter may record both atrial and ventricular activations. In some cases, the ventricular far field may interfere with atrial activation (e.g., signal interference), which may affect clinical understanding and interpretation of Carto maps. In accordance with one or more embodiments, the technical effects and benefits of the denoising autoencoder may include separating between ventricular far field and atrial based activation and generating separate maps for atrial and ventricular activation (e.g., the denoising autoencoder uses the model, during decoding, to separate between ventricular far field and atrial based activation within the one or more output intracardiac signals).

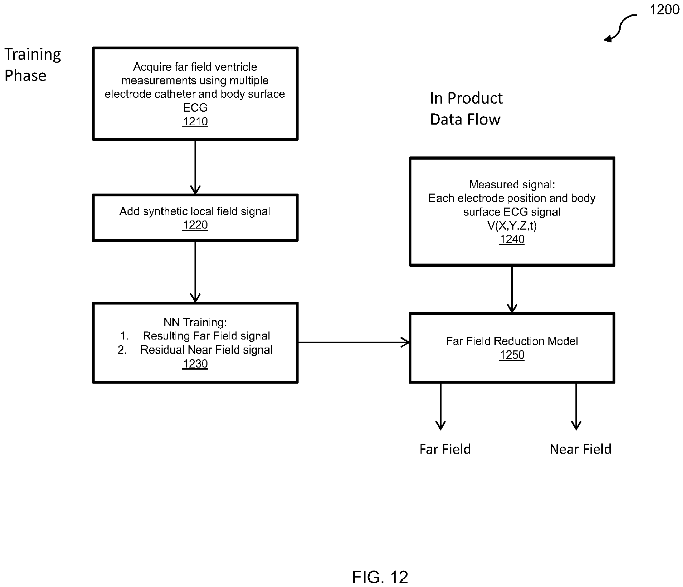

FIG. 12 is an example flow diagram of a method 1200 of decomposing near and far field signals in accordance with an embodiment. In a training phase, far field ventricle measurements are acquired (1210). These may be unipolar signals. The measurements may be acquired using a multiple electrode catheter and/or a body surface ECG. There may be numerous far field measurements. The far field measurements may be pure far field signals. In an embodiment, the pure far field signals may be from records where the bipolar signal is zero or almost zero. Therefore, near field in the unipolar signals may not exist, or be very small. In an embodiment, simulations may be used for the pure far field measurement by using, for example, a professional simulations software where pure far field signals may be generated. This may be done by controlling the sources that generate the ECG signals and using only far sources. In an embodiment, an expert may determine the pure far filed degree. In an embodiment, body surface ECG may contain mainly far field. In an embodiment, measurements from areas of scarred tissue may be used for pure far field measurements, which may not contain local activity and therefore near field is neglected.

Synthetic local field signals are added (1220). The synthetic local field signals may be for example from simulations of ECG signals. A large number of unipolar signals may be introduced in the training phase, so the algorithm may learn to recognize unipolar signal morphology. In addition, the algorithm may be exposed to far field signals and may be able to learn to detect the far field signals.

The algorithm may be configured to assess or learn both pure far field signals and a combination or mix of far and near field signals (real or synthetic mixed signals). The algorithm detects the far field component from the mixed signal, which is the communally part to all electrodes (far field). The near field is unique to the electrode, since it has a local activity that influences only small areas of the tissue, whereas the far field has a much greater contribution (both in signal magnitude and in dispersion within the tissue), and therefore it is called the communal part, since it is common to large number of electrodes. The communally part of the electrodes may be, and by subtraction of the far field component from the original signal, a pure near field signal.

Data may be collected routinely from multiple patients at EP procedures (1240) and provided to the system. The data may include regular unipolar signals, which are a combination of far and near field signals. These signals may be collected in any VT procedure using a multiple electrode catheter. Another approach may be to use synthetic signals that combine far field and near field components. For example, simulated or synthetic data may be used to generate pure far field signals that may serve as a gold standard. These signals may be generated using a professional simulations software or any other simulation program that may control the ECG signal sources. The data may include unique unipolar signals which include only a far field contribution without any near field components. This unipolar signal may be obtained by placing the catheter in a position without contacting the heart muscle. The signals may include ECG values and 3D location (per each electrode such that *signal* = *V(x, y, z, t).* The data may include body surface ECG signals, which are basically far field signals. The data may also include manual annotations of the unipolar signals identifying certain features of the underlying near field signal, in particular LATs. The body surface ECG signal data and/or the manual annotation data may be used to assist in training and/or validation of any DL model.

Before processing unipolar data to extract the near field contribution in the training phase (1230), a preprocessing filtering step may be performed to remove irrelevant signals and artifacts. The unipolar signals may be provided with manual annotation. The preprocessing filtering may be evaluated by a user (semi-automatic), while at a later stage the annotated data can be used to train a conventional classification convolutional neural network (CNN) to perform the filtering automatically.

The neural network training results with the far field signal estimation (1230). By knowing the far field contribution, the near field signal is the residual signal remaining after removal of the far field signal from the regular unipolar signal. A bipolar signal may then be reconstructed between two unipolar signal sets.

In the far field reduction model (1250), since it is known from the neural network training (1230) what the far field and near field signals look like, an autoencoder may automatically decompose the near and far field from the measured signals (1240).

The neural network may be implemented by a number of example approaches which could be applied to the decomposition or separation of far field and near field contributions (1250), including autoencoders and siamese networks.

Autoencoders (AEs) are a class of unsupervised DNNs which learn a reduced dimensionality representation of a given dataset, so that they are then able to produce new data which is statistically similar to the original dataset.

In the context of the current method, if a suitably selected AE is trained using EGM signals which contain only far field contributions without near field contributions, then it can be used to extract the far field contribution from any arbitrary EGM signal. The training phase aims to equalize the input X and the output X' (pure far field), while the prediction phase maps any arbitrary EGM signal to the far field signal which comprises it. There are several AEs types (for example Variational AEs, Reconstruction AEs, Denoising AEs, Adversarial AEs) that contain elements which may be utilized, for example.

Siamese networks, which comprise two identical parts, are trained in a fully supervised manner to differentiate between similar and dis-similar pairs of signatures. Then, when presented with a reference signature and a new signature, the network predicts whether the new signature is authentic or not (i.e. similar to the reference). In recent years the concept of Siamese networks has been generalized to DNNs, and successfully applied to face recognition and face verification. Recently, Siamese NN have been applied to unsupervised learning for visual representations and medical diagnostics. Siamese networks may be utilized due to the fact that two unipolar signals from very close electrodes (that form a bipolar pair) typically have very similar far field components. Thus, if two unipolar signals are fed to each part of a Siamese network, a cost function can be constructed that tends to equalize the output of the two parts. To avoid obtaining a trivial solution (such as identically zero signals), a constraining term can be added to the cost function. This is, for example, a term that tends to minimize the difference between the results and the average of the two input signals.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which includes one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. A computer readable medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire

Examples of computer-readable media include electrical signals (transmitted over wired or wireless connections) and computer-readable storage media. Examples of computer-readable storage media include, but are not limited to, a register, cache memory, semiconductor memory devices, magnetic media such as internal hard disks and removable disks, magneto-optical media, optical media such as compact disks (CD) and digital versatile disks (DVDs), a random access memory (RAM), a read-only memory (ROM), an erasable programmable read-only memory (EPROM or Flash memory), a static random access memory (SRAM), and a memory stick. A processor in association with software may be used to implement a radio frequency transceiver for use in a WTRU, UE, terminal, base station, RNC, or any host computer.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one more other features, integers, steps, operations, element components, and/or groups thereof.

The descriptions of the various embodiments herein have been presented for purposes of illustration, but are not intended to be exhaustive or limited to the embodiments disclosed. Many modifications and variations will be apparent to those of ordinary skill in the art without departing from the scope of the invention, which is defined by the claims. The terminology used herein was chosen to best explain the principles of the embodiments, the practical application or technical improvement over technologies found in the marketplace, or to enable others of ordinary skill in the art to understand the embodiments disclosed herein.

## Claims

1. A computer-implemented method (1100) of decomposing a near field signal and a far field signal, the method comprising:
in a training phase for an autoencoder:
acquiring (1210), by a training algorithm, far field ventricle measurements;
adding (1220) a synthetic local field signal; and
detecting (1250), by the training algorithm, a resulting far field signal and a residual near field signal;
receiving (1115) measured signals (1021);
encoding (1120), by the trained autoencoder, the measured signals to produce a latent representation; and
decoding (1130), by the trained autoencoder, the latent representation to decompose a near field component (1065) and a far field component (1054) from the measured signals.

2. The method of claim 1, wherein the signals are electrocardiograms (ECG) signals.

3. The method of claim 1, wherein the measured signals are unipolar signals.

4. The method of claim 1, wherein the far field ventricle measurements are acquired using a multiple electrode catheter (105) or a body surface ECG signal.

5. The method of claim 1, wherein the decoding the latent representation to decompose a near field component (1065) and a far field component (1054) is based on the detected resulting far field signal and a residual near field signal.

6. A system (161) comprising:
a memory storing processor executable instructions; and
a processor configured to execute the processor executable instructions to cause the system to perform the method of any preceding claim.

## Patentansprüche

1. Computerimplementiertes Verfahren (1100) zum Zerlegen eines Nahfeldsignals und eines Fernfeldsignals, wobei das Verfahren umfasst:
in einer Trainingsphase für einen Autocodierer:
Erfassen (1210) durch einen Trainingsalgorithmus von Fernfeld-Ventrikelmessungen;
Hinzufügen (1220) eines synthetischen Ortsfeldsignals; und
Detektieren (1250) durch den Trainingsalgorithmus eines resultierenden Fernfeldsignals und eines restlichen Nahfeldsignals;
Empfangen (1115) von gemessenen Signalen (1021);
Codieren (1120) durch den trainierten Autocodierer der gemessenen Signale, um eine latente Darstellung zu erzeugen; und
Decodieren (1130) durch den trainierten Autocodierer der latenten Darstellung, um eine Nahfeldkomponente (1065) und eine Fernfeldkomponente (1054) aus den gemessenen Signalen zu zerlegen.

2. Verfahren nach Anspruch 1, wobei die Signale Elektrokardiogramm(EKG)-Signale sind.

3. Verfahren nach Anspruch 1, wobei die gemessenen Signale unipolare Signale sind.

4. Verfahren nach Anspruch 1, wobei die Fernfeld-Ventrikel-Messungen unter Verwendung eines Mehrelektrodenkatheters (105) oder eines Körperoberflächen-EKG-Signals erfasst werden.

5. Verfahren nach Anspruch 1, wobei das Decodieren der latenten Darstellung, um eine Nahfeldkomponente (1065) und eine Fernfeldkomponente (1054) zu zerlegen, auf dem detektierten resultierenden Fernfeldsignal und einem restlichen Nahfeldsignal basiert.

6. System (161), umfassend:
einen Speicher, der durch einen Prozessor ausführbare Anweisungen speichert; und
einen Prozessor, der ausgelegt ist zum Ausführen der durch einen Prozessor ausführbaren Anweisungen, um zu bewirken, dass das System das Verfahren nach einem vorhergehenden Anspruch durchführt.

## Revendications

1. Procédé (1100) mis en œuvre par ordinateur de décomposition d'un signal en champ proche et d'un signal en champ lointain, le procédé comprenant :
dans une phase d'apprentissage pour un auto-encodeur :
l'acquisition (1210), par un algorithme d'apprentissage, de mesures de ventricule en champ lointain ;
l'ajout (1220) d'un signal en champ local synthétique ; et
la détection (1250), par l'algorithme d'apprentissage, d'un signal en champ lointain résultant et d'un signal en champ proche résiduel ;
la réception (1115) de signaux mesurés (1021) ;
l'encodage (1120), par l'auto-encodeur entraîné, des signaux mesurés pour produire une représentation latente ; et
le décodage (1130), par l'auto-encodeur entraîné, de la représentation latente pour décomposer une composante en champ proche (1065) et une composante en champ lointain (1054) à partir des signaux mesurés.

2. Procédé selon la revendication 1, les signaux étant des signaux d'électrocardiogrammes (ECG).

3. Procédé selon la revendication 1, les signaux mesurés étant des signaux unipolaires.

4. Procédé selon la revendication 1, les mesures de ventricule en champ lointain étant acquises en utilisant un cathéter à électrodes multiples (105) ou un signal d'ECG en surface corporelle.

5. Procédé selon la revendication 1, le décodage de la représentation latente pour décomposer une composante en champ proche (1065) et une composante en champ lointain (1054) étant basé sur le signal en champ lointain résultant et un signal en champ proche résiduel détectés.

6. Système (161) comprenant :
une mémoire stockant des instructions exécutables sur processeur ; et
un processeur configuré pour exécuter les instructions exécutables sur processeur pour amener le système à réaliser le procédé selon l'une quelconque des revendications précédentes.
